(19) Europäisches Patentamt
European Patent Office
Office européen des brevets

(11) Publication number: 0 269 230 A1

(12) # EUROPEAN PATENT APPLICATION

(21) Application number: 87308918.9

(22) Date of filing: 08.10.87

(51) Int. Cl.[4]: C07D 235/08 , C07D 235/24 , C07D 405/06 , A01N 43/52

(30) Priority: 09.10.86 GB 8624199
09.10.86 GB 8624200

(43) Date of publication of application: 01.06.88 Bulletin 88/22

(84) Designated Contracting States: AT BE CH DE ES FR GB GR IT LI LU NL SE

(71) Applicant: THE WELLCOME FOUNDATION LIMITED
183-193 Euston Road
London NW1 2BP(GB)

(72) Inventor: Weston, John Bernard
The Wellcome Research Laboratories
Ravens Lane
Berkhamsted Hertfordshire(GB)
Inventor: Pulman, David Allen
The Wellcome Research Laboratories
Ravens Lane
Berkhamsted Hertfordshire(GB)
Inventor: Frenkel, Alexander David
The Wellcome Research Laboratories
Ravens Lane
Berkhamsted Hertfordshire(GB)

(74) Representative: Rollins, Anthony John et al
Group Patents & Agreements The Wellcome Research Laboratories Langley Court
Beckenham Kent BR3 3BS(GB)

(54) **Heterocyclic compounds.**

(57) A compound of the formula (I)

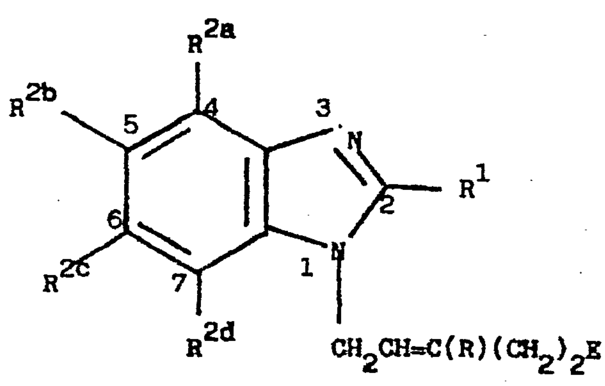

(I)

or an acid addition salt thereof, wherein R is hydrogen or $C_{1-4}$alkyl, $R^1$ is chlorine or methyl optionally substituted by one or two fluorine, $R^{2a}$ to $R^{2d}$ are the same or different and are chosen from the group comprising hydrogen, chlorine, bromine, fluorine, $C_{1-4}$ alkyl optionally substituted by fluorine or chlorine

EP 0 269 230 A1

atoms, $C_{1-4}$ alkoxy, $C_{1-4}$ acyl, $C_{1-4}$ alkoxycarbonyl, $C_{1-4}$alkylthio, nitro, cyano, amino optionally substituted by $C_{1-4}$ alkyl or $C_{1-4}$ acyl; E is $C_{6-10}$ aryl or aralkyl, a group $CH_2C(Me)_2OR^3$ wherein $R^3$ is hydrogen, $C_{1-4}$ acyl, $C_{1-4}$ alkyl or $C_{2-8}$ carbamyl, a group $CH=C(Me)CH_2CH_2CH=CMe_2$, a group - 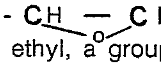, a group $OCHR^4R^5$ wherein $R^4$ and $R^5$ are the same or different and each is methyl or ethyl, a group $CH=CR^6R^7$ wherein $R^6$ is hydrogen, chlorine, bromine, fluorine, $C_{1-4}$ alkyl, $C_{1-8}$ alkoxycarbonyl, $C_{1-4}$alkynyl or $C_{1-8}$ acyl and $R^7$ is hydrogen, chlorine, bromine, fluorine or $C_{1-4}$ alkyl, or a group $CH=X\text{-}Y\text{-}R^8$ wherein X is N or $C(R^9)$ wherein $R^9$ is hydrogen or $C_{1-6}$alkyl, Y is oxygen, sulphur or a group $NR^{10}$ wherein $R^{10}$ is $C_{1-8}$ hydrocarbyl, $R^8$ is $C_{1-4}$ acyl or $C_{1-10}$ hydrocarbyl optionally substituted by $C_{1-4}$ alkyl, $C_{1-4}$ alkoxy, $C_{1-4}$ acyl or halogen, or, when X is nitrogen, $R^8$ may be hydrogen;
provided that at least of of $R^{2a}$ to $R^{2d}$ is other than hydrogen when $R^1$ is methyl or chlorine except when E is $OCHR^4R^5$, $C_{6-10}$ aryl and aralkyl, $CH=CR^6R^7$ or $CH=XYR^8$ or $R^3$ is $C_{2-8}$ carbamyl and that $R^{2c}$ is not methyl when $R^1$ is methyl except when E is $CH=CR^6R^7$ or $CH=XYR^8$, and other than 1-geranyl-2-methylbenzimidazole, processes for their preparation, formulations containing them and their use in the control of pests, helminths and fungi.

## HETEROCYCLIC COMPOUNDS

The present invention relates to a series of benzimidazole compounds, to their use, to compositions containing them, and to methods for synthesising them.

Eto (Agric.Biol.Chem., 46, 1715, 1982) has disclosed that benzimidazoles with a terpenoid moiety at the 1-position have insecticidal activity. However, he reported that substitution at the 5 or 6 position led to a drastic decrease in activity.

We have now found a series of related compounds having interesting acaracidal as well as insecticidal activity. Many of these compounds also have fungicidal and nematocidal activity.

Thus the present invention provides a compound of the formula (I):

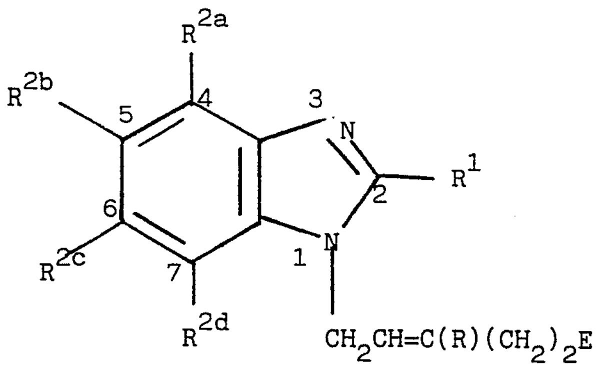

or an acid addition salt thereof, wherein R is hydrogen or $C_{1-4}$ alkyl, $R^1$ is chlorine or methyl optionally substituted by one or two fluorine, $R^{2a}$ to $R^{2d}$ are the same or different and are chosen from the group comprising hydrogen, chlorine, bromine, fluorine, $C_{1-4}$ alkyl optionally substituted by fluorine or chlorine atoms, $C_{1-4}$ alkoxy, $C_{1-4}$ acyl, $C_{1-4}$ alkoxycarbonyl, , $C_{1-4}$ alkylthio, nitro, cyano, amino optionally substituted by $C_{1-4}$ alkyl or $C_{1-4}$ acyl; E is $C_{6-10}$ aryl or aralkyl, a group $CH_2C(Me)_2OR^3$, wherein $R^3$ is hydrogen, $C_{1-4}$ acyl, $C_{1-4}$ alkyl, or $C_{2-8}$ carbamyl, a group CH = $CMeCH_2CH_2CH$ = $CMe_2$, a group $CH \overset{O}{=\!=} CMe_2$, a group $OCH\text{-}R^4R^5$ wherein $R^4$ and $R^5$ are the same or different and each is methyl or ethyl, a group $CH=CR^6R^7$ wherein $R^6$ is hydrogen, chlorine, bromine, fluorine, $C_{1-4}$ alkyl, $C_{1-8}$ alkoxycarbonyl. $C_{1-4}$ alkenyl or $C_{1-8}$ acyl and $R^7$ is hydrogen chlorine, bromine, fluorine or $C_{1-4}$ alkyl; or a group CH = X-Y-$R^8$, wherein X is nitrogen or C($R^9$) wherein $R^9$ is $C_{1-6}$ alkyl or hydrogen, Y is oxygen, sulphur or a group of $NR^{10}$ wherein $R^{10}$ is $C_{1-8}$ hydrocarbyl; $R^8$ is $C_{1-4}$ acyl or $C_{1-10}$ hydrocarbyl optionally substituted by $C_{1-4}$ alkyl, $C_{1-4}$ alkoxy, $C_{1-4}$ acyl or halogen, or when X is nitrogen, alternatively $R^8$ is hydrogen;

provided that at least one of $R^{2a}$ to $R^{2d}$ is other than hydrogen when $R^1$ is methyl or chlorine except when E is $OCHR^4R^5$, $C_{6-10}$ aryl and aralkyl, CH = $CR^6R^7$ or CH = $XYR^8$ or $R^3$ is $C_{2-8}$ carbamyl and that $R^{2c}$ is not methyl when $R^1$ is methyl except when E is $CH=CR^6R^7$ or CH = $XYR^8$, and provided that the compound of formula (1) is not 1-geranyl-2-methylbenzimidazole.

By hydrocarbyl is meant a hydrocarbon residue such as alkyl, alkenyl, alkynyl, aryl or alkyl, alkenyl or alkynyl substituted by aryl or aryl substituted by alkyl, alkenyl or alkynyl.

Preferably, R is hydrogen or methyl.

Advantageously the group $R^1$ is a methyl group, and preferably one of $R^{2a}$ to $R^{2d}$ is or are chosen from nitro, methyl, methoxy ethoxy and chlorine. Preferred groups E are $CH_2C(Me)_2OR^3$ wherein $R^3$ is preferably methyl, ethyl or acetyl; CH = $CMe_2$, CH = $CMeCH_2CH_2CH$ = $CMe_2$ and CH = $NOR^8$ wherein $R^8$ is $C_{1-4}$ alkyl or hydroegn preferably methyl or hydrogen.

Thus a particular group of compounds of formula I comprises a compound of formula (Ia)

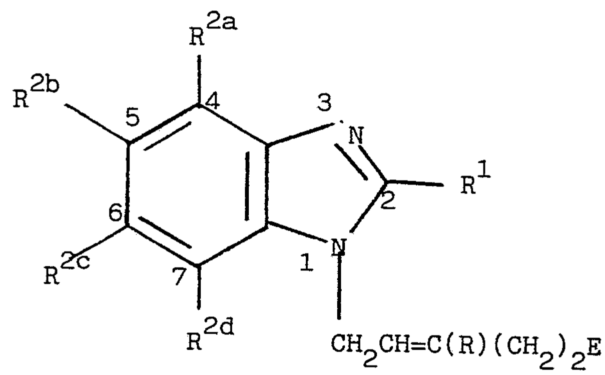

(1a)

or an acid addition salt thereof, wherein R is hydrogen or $C_{1-4}$ alkyl, $R^1$ is chlorine or methyl optionally substituted by one or two fluorine, $R^{2a}$ to $R^{2d}$ are the same or different and are chosen from the group comprising hydrogen, chlorine, bromine, fluorine, $C_{1-4}$ alkyl optionally substituted by fluorine or chlorine atoms, $C_{1-4}$ alkoxy, $C_{1-4}$ acyl, $C_{1-4}$ alkoxycarbonyl, , $C_{1-4}$ alkylthio, nitro, cyano, amino optionally substituted by $C_{1-4}$ alkyl or $C_{1-4}$ acyl; E is $C_{6-10}$ aryl or aralkyl, a group $CH_2C(Me)_2OR^3$, a group CH = $CMeCH_2CH_2CH$ = $CMe_2$, a group CH = $CMe_2$, a group $-CH\overset{O}{—}CMe_2$ or a group OCH-$R^4R^5$ wherein $R^4$ and $R^5$ are the same or different and each is methyl or ethyl, wherein $R^3$ is hydrogen, $C_{1-4}$ acyl, $C_{1-4}$ alkyl, or $C_{2-8}$ carbamyl provided that at least one of $R^{2a}$ to $R^{2d}$ is other than hydrogen when $R^1$ is methyl or chlorine except when E is OCH-$R^4R^5$ or $R^3$ is $C_{2-8}$ carbamyl and that one of $R^{2b}$ and $R^{2c}$ is not methyl when $R^1$ is methyl.

Another preferred group of compounds comprises a compound of formula (Ib):

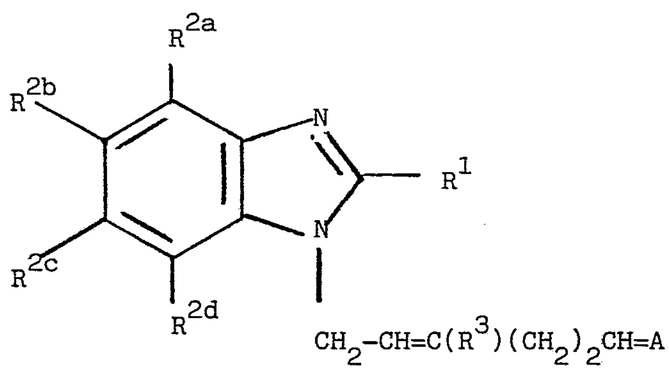

(Ib)

or an acid addition salt thereof, wherein $R^1$ is chlorine or methyl optionally substituted by one or two fluorine atoms; $R^{2a}$ to $R^{2d}$ are the same or different and are chosen from the group comprising hydrogen, chlorine, bromine, fluorine, $C_{1-4}$ alkyl optionally substituted by one or two fluorine or chlorine atoms, $C_{1-4}$ alkoxy, $C_{1-4}$ acyl, $C_{1-4}$ alkylthio, nitro, cyano, amino optionally substituted by $C_{1-4}$ alkyl or $C_{1-4}$ alkanoyl; R is hydrogen or $C_{1-4}$ alkyl; and A is a group X-Y-$R^8$ or a group $CR^6R^7$, wherein X is nitrogen, CH or $C(R^{9'})$ wherein $R^{9'}$ is $C_{1-6}$ alkyl, Y is oxygen, sulphur or a group $NR^{10}$ wherein $R^{10}$ is $C_{1-8}$ hydrocarbyl; $R^8$ is $C_{1-4}$ acyl or $C_{1-10}$ hydrocarbyl optionally substituted by $C_{1-4}$ alkyl, $C_{1-4}$ alkoxy, $C_{1-4}$acyl or halogen, or, when X is nitrogen, alternatively $R^8$ is hydrogen; $R^6$ is chosen from hydrogen, chlorine, bromine, fluorine, $C_{1-4}$ alkyl, $C_{1-8}$alkoxycarbonyl, $C_{1-4}$ alkenyl and $C_{1-8}$ acyl; and $R^7$ is chosen from hydrogen, chlorine, bromine, fluorine and $C_{1-4}$ alkyl, other than 1-geranyl-2-methylbenzimidazole.

Preferred compounds of the formula (I) include

2,4-Dimethyl-1-[(2E,6E)-3,7,11-trimethyldodeca-2,6,10-trienyl]benzimidazole
2,4,5-Trimethyl-1-[(2E,6E)-3,7,11-trimethyldodeca-2,6,10-trienyl]benzimidazole
(2E)-1-(7-Methoxy-3,7-dimethyloct-2-enyl)-2,4,5-trimethylbenzimidazole
(2E)-1-(7-Methoxy-3,7-dimethyloct-2-enyl)-2,4-dimethylbenzimidazole
2-Methyl-5(6)-methoxy-1-[(2E,6E)3,7,11-trimethyldodeca-2,6,10-trienyl]benzimidazole
5(6)-Chloro-1-geranyl-2-methylbenzimidazole
5(6)-Ethyl-1-geranyl-2-methylbenzimidazole
Methyl 1-geranyl-2-Methylbenzimidazole-7-carboxylate
4,6-Dichloro-1-geranyl-2-methylbenzimidazole
(E/Z)-1-(5-Isopropoxy-3-methylpent-2-enyl)-2-methylbenzimidazole

1-Geranyl-2,4-dimethylbenzimidazole
1-Geranyl-2,6,7-trimethylbenzimidazole
1-Geranyl-2-methyl-4-nitrobenzimidazole
1-Geranyul-5(6)-methoxy-2-methylbenzimidazole
4-Amino-1-geranyl-2-methylbenzimidazole
1-Geranyl-2-methyl-5(6)-nitrobenzimidazole
(5E)-7-(2-methylbenzimidazol-1-yl)-1,1,5-trimethylhept-5-enyl-N-phenylcarbamate
1-[(2E)-7,7-Dichloro-3-methylhepta-2,6-dienyl]-2-methylbenzimidazole
1-[(2E)-6-Methoxyamino-3-methylhex-2-enyl]-2-methylbenzimidazole
(6E)-6-Methyl-8-(2-methylbenzimidazole-1-yl)-1-phenylocta-2,6-dien-1-one
1-(3-Methylhepta-2,6-dienyl)-2-methylbenzimidazole
1-(Hepta-2,6-dienyl)-2-methybenzimidazole
(2E)-2-Methyl-1-(3-methylocta-2,6-dienyl)benzimidazole
2-Methyl-1-[(2E)-3-methyl-6-benzyloxyiminohex-2-enyl]benzimidazole
or acid addition salts thereof.

Compounds of the formula (I) exist as geometric isomers with respect to the double bond or bonds in the side chain attached to the 1-position of the benzimidazole ring. The present invention provides individual geometric isomers and mixtures thereof.

When the said side chain contains one or more chiral centres, the present invention includes all optical isomers and mixtures thereof.

The present invention also provides a process for the preparation of compounds of the formula (I), which process comprises

(a) the reaction of a compound of the formula (II):

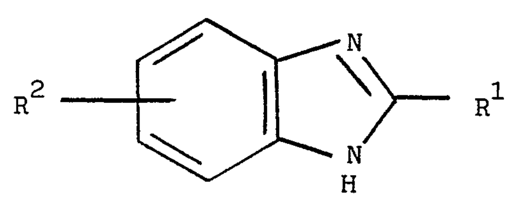

(II)

wherein $R^1$ is as hereinbefore defined and $R^2$ represents $R^{2a}$ to $R^{2d}$, with an alkylating agent of the formula $LCH_2$-$CH = C(R)(CH_2)_2E$ wherein L is a leaving group such as halogen or alkyl-or arylsuphonyloxy, or

b) when E is a group $CH = NOR^8$, $CH = CR^6R^7$, or $CH = CMeCH_2CH_2CH = CMe_2$, the reaction of a compound of the formula (III):

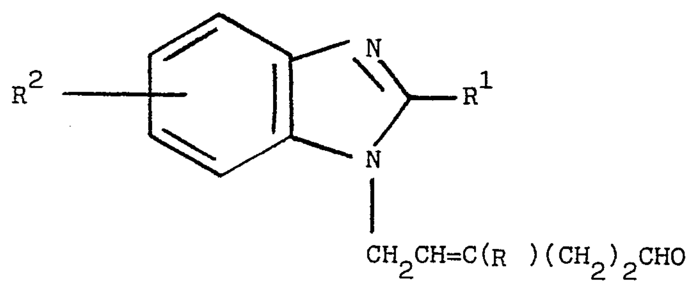

(III)

with the appropriate hydroxylamine or Wittig type reagent (for example $(R^{11})_3P = CR^6R^7$ or $(R^{11})_3P = CMeCH_2CH_2CH = CMe_2$ wherein $R^6$ and $R^7$ are as hereinbefore defined and $R^{11}$ is $C_{1-4}$ alkyl or $C_{6-10}$aryl, and therefore optionally converting one compound of the formula (I) into another compound of formula (I).

Reaction (a) is usually conducted in a polar solvent such as dimethylformamide or dimethylsulphoxide and in the presence of a base such as an alkali metal hydride (e.g. sodium hydride) or an alkali metal carbonate (e.g. potassium carbonate). The reaction is conducted at a non-extreme temperature, typically between -20°C and 150°C, usually at room temperature or with mild heating, i.e. between 25° and 50°C.

Compounds of the formula $L$-$CH_2CH = C(R)(CH_2)_2E$ and (II) are prepared according to methods well known in the art. Thus for example when E is a group $CH = NOR^8$, the alkylating agent may be prepared according to the following reaction sequence.

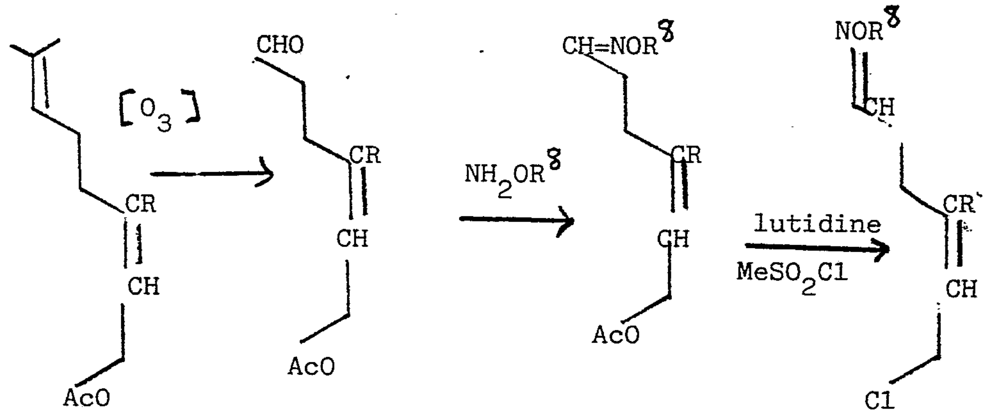


Process b) is conducted under conditions well knwn to those skilled in the art. Thus, when the desired end product has a group A = $NOR^8$, the compound of the formula (III) typically is reacted with $H_2NOR^8$ in a polar solvent at a non-extreme temperature, suitably -20°C to 150°C and preferably at room temperature.

Compounds of the formula (II) can be prepared by ozonolysis of compounds of the formula (IV):

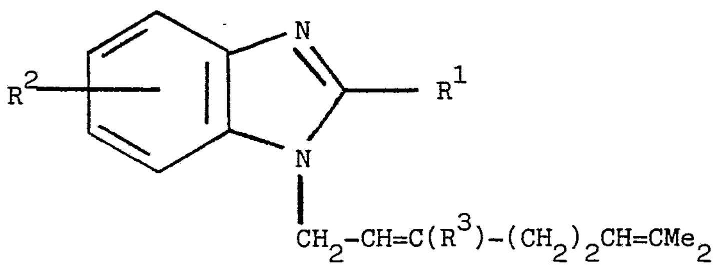

(IV)

which in turn can be prepared by reaction of compounds of the formula (II) with a geranyl halide, e.g. geranyl chloride, under conditions known in the art, see for example Kuwano et al. Agric. Biol. Chem ., 1982, 46, 1715-1716.

Compounds of the formula (III) can also be prepared by the hydrolysis of compounds of the formula (V):

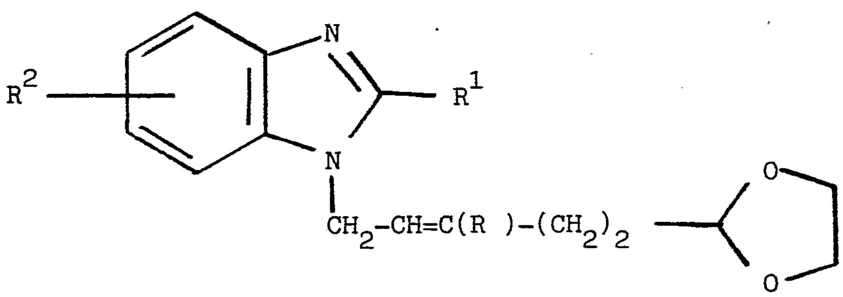

(V)

Compounds of the formula (V) can in turn be prepared by the reaction of a compound of the formula (II) with a compound

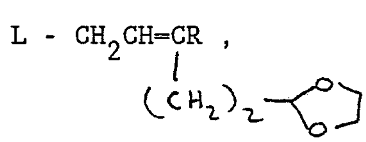

wherein L is a leaving group, according to standard methods.

Thus for example, compounds of the formula (II), wherein $R^1$ is methyl, can be prepared by reaction of a compound of the formula (VI):

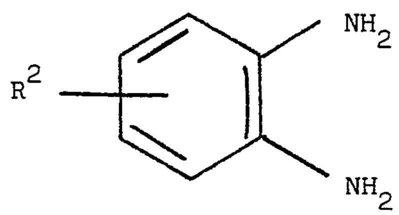

(VI)

with reactive derivatives of acetic acid, for example acetic anhydride.

When $R^1$ is chlorine, compounds of the formula (II) can be prepared by chlorination of a compound of the formula (VII):

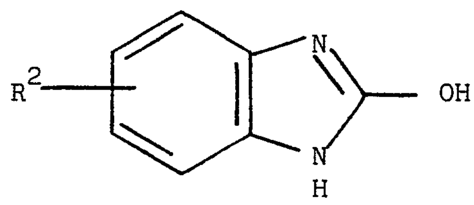

(VII)

according to well known methods; for example by treatment with phosphorus oxychloride.

Salts of the compounds of the formula (I) will be those formed by the addition of a suitable acid to a compound of the formula (I).

The compounds of formula (I) may be used to control or eradicate pests such as arthropods, e.g. insect and acarine pests; fungal pests, such as fungi harmful to crops, stored produce and wood; and helminths, e.g. animal helminths and soil nematodes. Thus, the present invention provides a method for the control or eradication of arthropods, fungal pests and/or helminths which comprises administering to the arthropod, fungal pest and/or helminth or to their environment an effective amount of a compound of the formula (I). The present invention also provides a method for the control or eradication or arthropod and/or helminth infestations of animals (including humans), and/or the control or eradication of arthropod, helminth and/or fungal infestations of plants (including trees) and/or stored products which comprises administering an effective amount of a compound of the formula (I). The present invention further provides for the compounds of the formula (I) for use in human and veterinary medicine, in public health control and in agriculture for the control of arthropod, fungal and/or helminth pests.

By the term "control" is meant the amelioration of present or future deleterious effects of pests and includes killing adults, larvae and eggs, the inhibition or reproduction, the repellency and/or knockdown of pests, and any other influence on behaviour.

Compounds of formula (I) are of particular value in the protection of field, forage, plantation, glasshouse, orchard and vineyard crops, of ornamentals and of plantation and forest trees; for example, cereals (such as maize, wheat, rice, millet, oats, barley, sorghum), cotton, tobacco, vegetable and salads (such as beans, cole crops cucurbits, lettuce, onions, tomatoes and peppers), field crops (such as potato, sugar beet, ground nuts, soyabean, oil seed rape), sugar cane, grassland and forage crops (such as lucerne), plantations (such as of tea, coffee, cocoa, banana, oil palm, coconut, rubber, spices), orchards and groves (such as of stone and pip fruit, citrus fruits, kiwifruit, mango, olives and walnuts), vineyards, ornamental plants, flowers and shrubs under glass and in gardens and parks, forest trees (both deciduous and evergreen) in forests, plantations and nurseries and plants grown for industrial or pharmaceutical purposes (such as the evening primrose).

They are also valuable in the protection of timber (standing, felled, converted, stored or structural) from attack by sawflies (e.g. Urocerus) of beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

Compounds of formula (I) are of value in the control of public health pests, for example cockroaches and ants.

Compounds of formula (I) are also of value in the control of arthropods or helminths which are injurious

to, or spread or act as vectors of diseases in man and domestic animals, for example those hereinbefore mentioned, and more especially in the control of ticks, mites, lice, fleas, midges, biting, nuisance and myiasis flies, mosquitos and hemiptrean bugs.

Compounds of formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapour emanator (e.g. vapourising mat), combustible coil, bait, dietry supplement, wettable powder, granule, cream, ointment, aerosol, emulsifiable concentrate, oil suspension, oil solution, pressure-pack, impregnated article, (such as an ear tag or collar) microcapsule, pour-on formulation or other standard formulations well known to those skilled in the art. Sprays may be applied by hand or by means of a spray race or arch or by vehicle or aircraft mounted apparatus. The animal, soil, plant or other surface being treated may be saturated with the spray by means of high volume application or supoerficially coated with the spray by means of light or ultra low volume application. Dip concentrates are not applied per se, but diluted with water and the animals immersed in a dipping bath containing the dip wash. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

Compounds of fomula (I) may be prepared either as formulations ready for use on the animals, plants or surface or as formulations requiring dilution prior to application, but both types of formulation comprise a compound of formula (I) in intimate admixture with one or more carriers or diluents. The carriers may be liquid, solid or gaseous or comprise mixtures of such substances, and the compound of formula (I) may be present in a concentration of from 0.025 to 99% w/v depending upon whether the formulation requires further dilution.

Dusts, powders and granules and other solid formulations comprise the compound of formula (I) in intimate admixture with a powdered solid inert carrier, for example suitable clays, kaolin, bentonite, attapulgite, adsorbent carbon black, talc, mica, silica, chalk, gypsum, tricalcium phosphate, powdered cork, magnesium silicate, vegetable carriers, starch and diatomaceous earths. Such solid formulations are generally prepared by impregnating the solid diluents with solutions of the compound of formula (I) in volatile solvents, evaporating the solvents and, if desired, grinding the products so as to obtain powders and, if desired, granulating, compacting or encapsulating the products.

Sprays of a compound of formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 99.5 w/v of the composition and may be selected from kerosene, ketones, alcohols, xylene, aromatic naphtha, water, mineral oil, aromatic and aliphatic esters, and other solvents known in the formulating art. The concentration of emulsifiers may be varied within wide limits but is preferably in the range of 3 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates, soaps, lecithins, hydrolysed glues, etc..

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 0.5 to 99.5% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of formula (I) in intimate admixture with a dispersing agent and one or more surface active agents.

Aqueous suspensions of a compound of formula (I) may comprise a suspension in water together with suspending, stabilizing or other agents. Aqueous solutions may also be formed from acid addition salts of a compound of formula (I). The suspensions or solutions may be applied per se or in a diluted form in known

fashion.

Wetting agents and/or solubilisers may also be present in formulations of the present invention if appropriate or required.

Greases (or ointments) may be prepared from vegetable oils, synthetic esters of fatty acids or wool fat together with an inert base such as soft paraffin. A compound of formula (I) is preferably distributed uniformly through the mixture in solution or suspension. Greases may also be made from emulsifiable concentrates by diluting them with an ointment base.

Pastes and shampoos are also semi-solid preparations in which a compound of formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of formula (I) required for treatment.

Aerosol sprays may be prepared as a simple solution of the active ingredient in the aerosol propellant and co-solvent such as halogenated alkanes, propane, butane, dimethyl ether and the solvents referred to above, respectively. Pour-on formulations may be made as a solution or suspension of a compound of formula (I) in a liquid medium. An avian or mammal host may also be protected against infestation of acarine ectoparasites by means of carrying a suitably-moulded, shaped plastics article impregnated with a compound of formula (I). Such articles include impregnated collars, tags, bands, sheets and strips suitably attached to appropriate parts of the body. Suitably the plastics material is a polyvinyl chloride (PVC). Impregnated bands, sheets or strips may also be placed in a suitable position in a room. animal house or other suitable environment to control pesticidal infestation of that environment.

The concentration of the compound of formula (I) to be applied to a locus e.g. an animal, premises, other substrates or outdoor areas will vary according to the compound chosen, the interval between treatments, the nature of the formulation, the likely infestation and the nature of the locus, but in general 0.001 to 20.0% w/v and preferably 0.01 to 10% of the compound should be present in the applied formulation. The amount of the compound deposited will vary according to the compound chosen, the method of application, area of application, concentration of the compound in the applied formulation, factor by which the formulation is diluted and the nature of the formulation, but will generally lie in the range of from 0.0001% to 4% except for undiluted formulations.

Undiluted formulations such as pour-on formulations in general will be applied at a concentration in the range from 0.1 to 20.0% w/w and preferably 0.1 to 10%. The amount of compound to be applied to stored products in general will lie in the range of from 0.1 to 20ppm. Space sprays may be applied to give an average initial concentration of 0.001 to 1mg of compound of formula (I) per cubic metre of treated space.

Compounds of formula (I) are of use in the protection and treatment of plant species, in which case an effetive insecticidal, acaricidal, fungicidal or nematocidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (I) and conveniently between 0,1 and 15% by weight of a compound of the formula (I).

Dusts, greases, pastes and aerosol formulations are usually applied in a random fashion as described above and concentrations of 0.001 to 90% w/v preferably 0.001 to 20% w/v of a compound of formula (I) in the applied formulation may be used.

Compounds of formula (I) have been found to have activity against the common housefly (Musca domestica) and against the wood destroying fungi: brown rot (Coniophora puteana), white rot (Coriolus - (Trametes) versicolor), staining (Aureobasidium pallulans) and soft rot (Chaetomiumglobosum). In addition, certain compounds of formula (I) have activity against other arthropod pests including Blattella germanica and Boophilus microplus. The compounds of formula (I) are thus useful in the control of arthropods and fungi in any environment where these constitute pests, e.g. in agriculture, in animal husbandry, in public health control and in domestic situations.

Insect pests include members of the orders Coleoptera (e.g. Anobium,Ceutorhynchus,Rhynchophorus, Hylotrupes, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitpohilus, Diabrotica, Anthonomus or Anthrenusspp.), Lepidoptera (e.g. Ephestia, Tinea, Mamestra, Earias, Pectinophora, Ostrinia, Trichoplusia, Pieris, Laphygma, Agrotis, Amathes, Wiseana, Tryporyza, Diatraea, Sporganothis, Cydia, Archips, Plutella, Chilo, Heliothis, Spodoptera or Tineola spp.), Diptera (e.g. Musca , Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma , Hylemyia, Atherigona, Chlorops , Phytomyza, Ceratitis,

Liriomyza and Melophagus spp.), Phthiraptera ( Malophaga e.g. Damalina spp. and Anoplura e.g. Linognathus and Haematopinus spp.), Hemiptera (e.g. Aphis, Bemisia,Phorodon , Aeneolamia, Empoasca, Parkinsiella, Pyrilla, Aonidiella, Coccus, Pseudococcus, Helopeltis, Lygus, Dysdercus, Oxycarenus, Nezara, Aleurodes, Triatoma,Rhodnius, Psylla, Myzus, Megoura , Phylloxera, Adelyes, Niloparvata Nephrotettix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta , Lasius, Solenopsis or Monomorium spp.), Isoptera (e.g. Odontotermes and Reticulitermes spp.), Siphonaptera (e.g. Ctenocephalides or Pulex spp.), Thysanura (e.g. Lepsima spp.), Dermaptera (e.g. Forficula spp.), Psocoptera (e.g. Peripsocus spp.) and Thysanoptera (e.g. Thrips tabaci),.

Acarine pests include ticks, e.g. members of the genera Boophilus,Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes , Eurombicula, Demodex, Panonychus, Bryobia and Eriophyes Dermatophagoides, Tyrophagus and Glycyphagus .

Nematodes which attack plants and trees of importance to agriculture, forestry, horticulture, either directly or by spreading bacterial, viral mycoplasma of fungal diseases of the plants, include root-knot nematodes such as Meloidogyne spp. (e.g. M. incognita); cyst nematodes such as Globodera spp. (e.g. G. rostochiensis); Heteroderaspp. (e.g. H. avenae); Radopholus spp. (e.g. R. similis); lesion nematodes such as Pratylenchus spp. (e.g. P. pratensis ); Belonolaimus spp. (e.g. B. gracilis); Tylenchulus spp. (e.g. T. semipenetrans); Rotylenchulus spp. (e.g.R . reniformis); Rotylenchus spp. (e.g. R. robustus); Helicotylenchus spp. (e.g. H. multicinctus);Hemicycliophora spp. e.g. H. gracilis); Criconemoides spp. (e.g. C. similis); Trichodorus spp. (e.g. T primitivus) ; dagger nematodes such as Xiphinema spp. (e.g. X diversicaudatum), Longidorus spp (e.g. L. elongatus); Hoplolaimus spp. e.g. H. coronatus); Aphelenchoides spp. (e.g. A. ritzema-bosi, A. besseyi); stem and bulb eelworms such as Ditylenchus spp. (e.g. D. dipsaci).

Fungal pests includes wood destroying and crop fungi such as Basisomycetes e.g. Merulius lacrymans, Poria placenta, Gloeophyllum spp., Coriolus (Trametes) versicolor and Stereum hirsutum; Puccinia recondita , Uromyces phasioli, Ustilago maydis, Tilletia caries, Ustilago nuda, Rhizoctonia solani; Ascomycetes, e.g. Chaetomium globosum, Venturia inaequalis, Sphaerotheca fuliginea, Podosphaera leucotricha, Erysiphe graminis, Uncinula necator, Pyrenophora spp.; Deuteromycetes , e.g. Aureobasidium pullulans, Fusarium culmorum, Septoria nodorum, Botrytis fabae, Piricularia oryzae, Botrytis cinerae, Fusarium nivale, Verticillium albo-atrum and Penicillium digitatum; Mastigomycetes , e.g. Phytophthora spp. Plasmopara viticola and Pythium ultimum.

The compounds exhibit killing and/or knockdown activity against arthropod pests, and can be used to control larval pests as well as adult pests.

Compounds of the invention may be combined with one or more other active ingredients (for example insecticides (such as pyrethroids, carbamates, lipid amides and organophosphates), fungicides, anthelmintics and/or antimicrobial agents (see for example European Patent Applicatrion 148 526, page 6, line 32 to page 7, line 19 which is incorporated by reference herein) and/or with attractants, repellents and the like. Furthermore, the activity of the compounds of the invention may be enhanced by the addition of a synergist or potentiator, for example: one of the oxidase inhibitor class of synergists, such as piperonyl butoxide or propyl 2-propynylphenylphosphonate; a second compound of the invention; or a pyrethroid pesticidal compound. When an oxidase inhibitor synergist is present in a formula of the invention, the ratio of synergist to compound of formula (I) will be in the range 25:1-1-:25 e.g. about 10:1.

Stabilisers for preventing any chemical degradation which may occur with the compounds of the invention include, for example, antioxidants (such as tocopherols,· butylhydroxyanisole and butylhydroxytoluene) and scavengers (such as epichlorhydrin).

The following Examples illustrate, in a non-limiting manner, preferred aspects of the invention.

PROCESS A

(5E)-7-Acetoxy-1,1-dichloro-5-methylhepta-1,5-diene

A solution of (4E)-6-acetoxy-4-methyl-hex-4-enal (0.8g) and bromotrichloromethane (0.94g) in dry dichloromethane (14ml) was cooled to -20°C and stirred under nitrogen while a solution of hexamethylphosphoroustriamide (1.6g) in dichloromethane (10ml) was added dropwise. After 15 mins water and

hexane were added and the solutions separated. The organic solution was washed with dilute hydrochloric acid, water, brine, dried and evaporated. The crude product was column chromatographed on silica eluting with 1:1 ether : hexane. The product (250mg) has n.m.r. peaks at δ1.8(s,3H,$CH_3$); 2.0(s,3H,$COCH_3$); 2.2-(m,4H,$CH_2CH_2$); 4.5(d,2H,$NCH_2$,8Hz); 5.2(t,1H, = CH,8Hz); 5.6(t,1H, = CH,6Hz).

(2E)-7,7-Dichloro-3-methylhept-2,6-diene-1-ol

The above ester (250mg) and anhydrous potassium carbonate (0,2g) in methanol (20ml) was stirred at room temperature for 4 h and evaporated to dryness. Water and ether were added and the solutions separated. The ethereal solution was washed with brine, dried and evaporated to give the product (150mg), n.m.r. peaks at δ1.6(s,3H,$CH_3$); 2.2(m,4H,$CH_2CH_2$); 4.0(d,2H,$NCH_2$,8Hz), 5.3(t,1H, = CH, 8Hz); 5.6-(t,1H, = CH,6Hz).

(2E)-1,7,7-Trichloro-3-methylhept-2,6-diene.

The above alcohol (150mg) and lutidine (99mg) was stirred while anhydrous lithium chloride (33mg) in dimethylformamide (4ml) was added. The mixture was cooled to 0°C and mesylchloride (77μl) added and stirring continued overnight at this temperature. Water and ether were added and the ethereal solution separated and washed with saturated cupric nitrate solution, diluted with water water and brine, dried and evaporated yielding the product 150mg) with n.m.r. peaks at δ 1.8(s,3H,$CH_3$); 2.1(m,4H,$CH_2CH_2$); 4.0-(d,2H,$NCH_2$,8Hz); 5.4(t,1H = $CH_2$,8Hz), 5.6(t,1H, = $CH_2$,6Hz)

PROCESS B

(2E)-1-(6-Ethylenedioxy-3-methyl-hex-2-enyl)-2-methylbenzimidazole

2-Methylbenzimidazole was alkylated as for Process A using 6-ethylenedioxy-1-bromo-3-methylhex-2-(E)-ene to give the product which had n.m.r. peaks at δ1.8(s,3H,Me); 2.0(m,4H,$CH_2CH_2$); 2.4(s,3H,N = CMe); 3.8(m,4H,$OCH_2CH_2O$); 4.6 (d,2H,$NCH_2$, 8Hz); 5.2(t,1H, = CH,8Hz); 7.0-7.6(m,4H, aromatics).

4-Methyl-6-(2-methylbenzimidazole-1-yl)hex-4-enal

The above acetal (100mg) dissolved in ethyl acetate (10ml) was shaken with conc hydrochloric acid (3ml) for 15 min. A solution of 2N sodium hydroxide was added and the organic phase separated washed with water, brine, dried and evaporated to give the aldehyde (45mg) n.m.r. peaks at δ1.8(s,3H,Me); 2.0-(m,4H,$CH_2CH_2$); 2.6(s,3H, N = CMe); 4.6(d,2H,$NCH_2$,8Hz); 5.0(t,1H, = CH,8Hz); 7.1-7.8(m,4H, aromatics); 9.4-(t,1H,CHO, 2Hz).

PROCESS C

4-Methyl-6-(2-methylbenzimidazol-1-yl)hexa-4-enal

A solution of 1-geranyl-2-methylbenzimidazole (1.0g) in methanol (100ml) was cooled to -70°C and selectively ozonized. Dimethylsulphide (2ml) was added and the solution stirred at room temperature overnight. The solvent was removed and water and ethyl acetate added. The organic solution was separated, washed with brine, dried and evaporated to give the aldehyde (200 mgs).

EXAMPLE 1

1-, (2E)-7,7-Dichloro-3-methylhepta-2,6-dienyl,-2-methylbenzimidazole.

2-Methylbenzimidazole (100mg) in dry diemthylformamide (4ml) was added to a suspension of sodium hydride (30mg, 60% dispersion in oil) in dimethyl formamide (1ml) stirred under nitrogen at 0°C. After 0.5h 1,7,7-trichloro-3-methylhept-2E,6-diene (150mg) (from example A) in dimethylformamide (1ml) was added and the mixture left to stand at room temperature overnight. Ice and water were added and the mixture extracted with ethylacetate. The organic phase was washed with brine, dried and the solvents removed "in vacuo". Chromatography on silica eluting with ethyl acetate yielded the product (52mg), $n_D$ = 1.5446, with n.m.r. peaks at δ1.8(s,3H,Me);2.2(m,4H,$CH_2CH_2$);2.5(s,3H,$NCH_3$); 4.6(d, 2H, $NCH_2$, J=8Hz); 5.2(t,1H,=CH,8Hz); 5.6(t, 1H, =CH,6Hz); 7.0-7.7(m,4H, aromatics).

EXAMPLE 2

1-[(2E)-6-Methoxyimino-3-methylhex-2-enyl]-2-methybenzimidazole

A solution of aldehyde from process C (200mg) in tetrahydrofuran (15ml) was treated successively with methoxylamine hydrochloride (200mg) and pyridine (200µl) and water (4ml). The solution was heated at reflux for 1h, cooled and evaporated. Chromatography (silica) eluting with ethyl acetate yielded the product (52mg).

EXAMPLE 3

1-[(2E)-6-methoxyimino-3-methylhex-2-enyl]2-methybenzimidazole

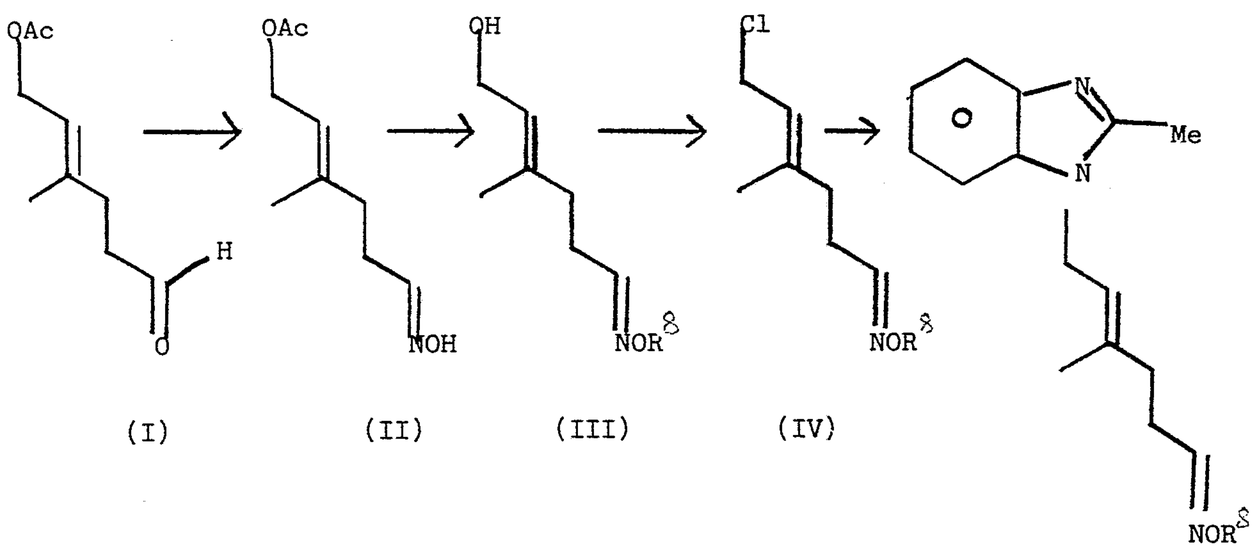

(I) (II) (III) (IV)

Oxime (II)

6-Acetoxy-5-methyl-trans-4-hexenal (5g) dissolved in tetrahydrofuran (100ml) was stirred while a solution of hydroxylamine hydrochloride (4g) in water (25ml) and pyridine (7ml) was added. The mixture was heated at reflux for 1h, cooled and water and ether added. The organic solution was separated and washed with dilute hydrochloric acid, saturated sodium bicarbonate, brine, dried and evaporated yielding the oxime (II) (5.1g), n.m.r. peaks at δ1.8(s,3H,COMe); 2.1(s,3H,Me); 2.4(m,4H,$CH_2CH_2$); 4.7(d,2H,$CH_2$,O); 5.4-(t,1H,=CH); 6. and 7.4(t,1H,HC=N).

Oxime (III)

Anhydrous potassium carbonate (5g) and iodomethane (2.3g) were added to a solution of oxime (II) (2g) in dry ethanol (60ml) and the mixture heated at reflux for 3h. The solvent was removed "in vacuo" and ether and water were added. The organic solution was separated and washed with water, brine, dried and concentrated to give oxime (III) (1.26g), n.m.r. peaks at δ1.8(s,3H,Me); 2.3(m,4H,$CH_2CH_2$); 3.9(s,3H,OMe); 4.2(d,2H,$CH_2$,O); 5.5(t,1H, =CH), 6.8 and 7.4(t,1H,HC=N).

A number of analogues of oxime (III) were prepared whose $^1$H n.m.r. were similar except where stated:

$R^8$ = benzyl, 4.6 and 5.0(s,2H,$CH_2$Ph); 7.1(s,5H,aromatics).

$R^8$ = propargyl, 1.3(m,1H, ≡CH); 4.7(d,2H,$CH_2$C≡).

$R^8$ = allyl, 4.6 and 5.3(d,2H,$CH_2$C=); 5.2(m,2H,$CH_2$=).

$R^8$ = n-butyl, 1.0-2.0(m,7H,$CH_2CH_2CH_3$); 4.0 and 4.1(m,2H,$CH_2$O)

$R^8$ = ethyl, 1.3(t,3H,$CH_2\underline{CH_3}$); 4.1(q,2H,$\underline{CH_2}CH_3$),

Chloride (IV) $R^8$=Me

Lithium chloride (370mg) was added to a solution of oxime (III) (1.2g) and lutidine (0.85g) in dimethyformamide (10ml). The mixture was cooled to 0°C and methyl sulphonyl chloride (0.9g) was added. After standing at -10°C overnight water and ether were added and the ethereal solution separated and washed with water, saturated sodium chloride, dried and concentrated to give the product (0.5g). n.m.r. peaks at δ1.8(s,3H,Me); 2.3(m,4H,$CH_2CH_2$); 3.9(s,3H,OMe); 4.0(d,2H,$CH_2$); 5.5(t,1H=CH), 6.8 and 7.4-(t,1H,HC=N).

1-[(2E)-6-methoxyimino-3-methylhex-2-enyl)-2-methylbenzimidazole

2-Methylbenzimidazole (200mg) was alkylated with chloride (iv) (300mg) as described in example 4. The product had n.m.r. peaks at δ1.9(s,3H,Me); 2.3(m,4H,$CH_2CH_2$); 2.6(s,3H,N=C-Me); 3.75 and 3.85(s,3H, OMe); 5.2(t,2H,$CH_2$N); 6.5(t,1H,CH=N); 7.2-7.6(m,4H,aromatic). A number of analogues were prepared whose $^1$H n.m.r. were similar except where stated.

2-Methyl-1-[(2E)-3-methyl-6-benzyloxyiminohex-2-enyl]benzimidazole

R=benzyl, 5.4(s,2H,$\underline{CH_2}$ Ph); 7.2(s,5H,aromatics).

2-Methyl-1-[(2E)-3-methyl-6-(prop-2-ynyloxyimino)hex-2-enyl]benzimidazole.

R=propargyl, 1.3(m,1H, ≡CH); 4.6 (d,2H,$\underline{CH}_2$O).

1-[(2E)-Butoxyimino-3-methylhex-2-enyl]-2-methylbenzimidazole.

R=n-butyl, 1.0-2.0 (m,7H, $CH_2CH_2CH_3$); 4.1 (m,2H, $OCH_2$.)

1-[(2E)-6-Ethoxyimino-3-methylhex-2-enyl]-2-methylbenzimidazole.

R=ethyl, 1.0 and 1.1 (t, 3H, $CH_2\underline{CH_3}$); 3.9 and 4.0 (q,2H,$\underline{CH_2}CH_3$).

EXAMPLE 4

1-[(2E)-7-methylocta-2,6-dienyl]-2-methylbenzimidazole

a) Ethyl-7-Methyloct-2,6-dienoate

n-Butyl lithium (1.6M in hexane) (5.1 ml) was added to a solution of diisopropylamine (0.67 g) in dry tetrahydrofuran (30 ml) stirred at 0°C under nitrogen. The solution was cooled to -78°C and triethylphosphonoacetate (1.8 g) in tetrahydrofuran (10 ml) was introduced. After stirring for a further 40 minutes a solution of 5-methylhex-4-enal (1.8 g) in tetrahydrofuran (5 ml) was added and the solution allowed to warm to room temperature overnight. Ether and water were added and the ethereal solution separated, washed with dilute hydrochloric acid, saturated sodium-bicarbonate solution and, brine, dried over sodium sulphate and evaporated to yield the product (2.1 g), $n_D$ = 1.4610, infrared (liquid film) 1730 $cm^{-1}$.

b) 7-Methyloct-2,6-dien-1-ol

The above ester (1.0 g) dissolved in dry benzene (20 ml) was cooled to 5°C and a solution of sodium bis (2-methoxyethoxy) - aluminium hydride (0.8 ml) in benzene (10 ml) was added over 15 minutes. The solution was allowed to warm to room temperature overnight and saturated ammonium chloride solution was added. The organic phase was separated and washed with brine, dried over sodium sulphate and evaporated to give the product (0.7 g), $n_D$ = 1.4770, infrared (liquid film) 3000-3500 $cm^{-1}$.

c) 7-Methyloct-2,6-dienyl bromide

A solution of the above alcohol (0.7 g) in diethyl ether (10 ml) was cooled to 0°C and treated with pyridine (100ml) and phosphorus tribromide (200 µl). The mixture was allowed to warm to room temperature over 3 hours and ice added. The ethereal solution was separated and washed with water, saturated sodium-bicarbonate solution, brine, dried over sodium sulphate and evaporated yielding an oil (0.7 g), $n_D$ = 1.4978, nmr peaks at δ 1.0 (m, 6H, 2xMe); 2.1 (m, 4H, $CH_2CH_2$); 4.0 (m, 2H, $NCH_2$); 5.1 (m, 1H, = CH); 5.7 (m, 2H, CH = CH).

d) 1-[(2E)-7-methylocta-2,6-dienyl]-2-methylbenzimidazole

2-Methylbenzimidazole was alkylated with 7-methyloct-2,6-dienyl bromide as for Example 1 to give an oil $n_D$ 1.5550, nmr peaks at δ 1.5 and 1.6 (s, 6H, 2xMe); 2.0 (m, 4H, $CH_2CH_2$); 2.5 (s, 3H, =NMe); 2.5 (m, 2H, $NCH_2$); 5.0 (m, 1H, =CH); 5.4 (m, 2H, CH=CH); 7.1 (m, 4H, aromatics).

EXAMPLE 5

(4E)-4-Methyl-6-(2-methylbenzimidazol-1-yl)hex-4-enal oxime acetate

Acetyl chloride (90 ml) was added to a stirred solution of (4 E)-4-methyl-6-(2-methylbenzimidazol-1-yl) hex-4-enal oxime (150 mg) and sodium hydroxide (52 mg) in water (2 ml). After stirring overnight at room temperature ethyl acetate was added. The organic solution was separated and washed with water, brine, dried over sodium sulphate and evaporated "in vacuo" to yield the product (50 mg). nmr peaks at δ 1.9 (s, 3H, Me); 2.1 and 2.2 (S, 3H, 2xCOMe); 2.4 (m, 4H, $CH_2CH_2$); 2.6 (S, 3H, =NMe); 4.7 (m, 2H, $NCH_2$); 5.2 (m, 1H, =CH); 7.2 (m, 4H, aromatics).

EXAMPLE 6

(7E)-7-Methyl-9-(2-methylbenzimidazole-1-yl)nona-3,7-dien-2-one

A solution of the aldehyde from process C (200mg) and acetylmethylenetriphenylphosphorane (530mg) in benzene (20mls) was heated at reflux for 6h. After cooling water and ether were added and the mixture separated. The ethereal solution was washed with brine, dried and evaporated. Chromatography (silica) eluting with 1:10; hexane: ethyl acetate yielded the product (70 mg) n.m.r. peaks at δ 1.8(s,3H,Me); 2.1-

(s,3H,COMe); 2.2(m,4H,$CH_2$ $CH_2$); 2.4(s,3H, N=CMe); 4.6(m,2H,$NCH_2$); 5.2(m,1H,=CH); 6.0(d,1H,=CH, 16Hz); 6.6(m, 1H, =CH); 7.2(m,4H, aromatics).

The following compounds were prepared in a manner analogous to that described above by reacting the aldehyde from Example C with the appropriate Wittig reagent:

(6E)-6-Methyl-8-(2-methylbenzimidazol-1-yl)-1-phenylocta-2,6-dien-1-one

nmr peaks at δ1.8 (S, 3H, Me); 2.3 (m, 4H, $CH_2CH_2$); 2.5 (s, 3H, N=CMe); 4.6 (m, 2H, $NCH_2$); 5.2 (m, 1H, =CH); 6.8-8.0 (m, 11H, aromatics + CH=CHO).

Ethyl 6-methyl-8-(2-methylbenzimidazol-1-yl)octa-2,6-dienoate

nmr peaks at δ1.2 (t, 3H, $CH_2Me$,7Hz); 1.8 (s, 3H, Me); 2.3 (m, 4H, $CH_2CH_2$); 2.4 (s, 3H, N=CMe); 4.2 (q, 2H, $OCH_2$, 7Hz); 4.6(m, 2H, $NCH_2$); 5.2 (m, 1H, =CH); 7.1-7.9 (m, 6H, aromatics + CH=CHCO).

(2E)-2-Methyl-1-(3-methlocta-2,6-dienyl)benzimidazole

$n_D$ = 1.5085 nmr peaks at δ1.4 (m, 3H, =CHMe); 1.7 (s, 3H, Me); 2.0 (m, 4H, $CH_2CH_2$); 2.4 (s, 3H, N=CMe); 4.4 (m, 2H, $NCH_2$); 5.1 (m, 3H, =CH); 7.2 (m, 4H, aromatics).

Example 7

2-Chloro-1-geranylbenzimidazole

2-Chlorobenzimidazole (0.4g) in dry dimethylformamide (10ml) was added to a suspension of sodium hydride (0.13g, 50% dispersion in oil) in dimethylformamide (2ml) stirred under nitrogen at 0° C. After 0.5h a solution of geranyl chloride (0.44g) in dimethylformamide (1ml) was added and the mixture left to stir at room temperature overnight. Ice and water were added and the mixture extracted with ethyl acetate. The organic phase was washed with brine, dried and the solvents removed. The crude material was purified by column chromatography (silica, ethyl acetate) yielding an oil $n_D$ = 1.5555 nmr peaks at 1.6, 1.7 and 1.9(s, 9H, 3 x Me); 2.0(m, 4-H, $CH_2$ $CH_2$); 4.7(d, 2H, NC<u>H</u> 2, 8Hz); 5.0(m, 2H, = CH x 2), 7.0-7.6(m, 4H, aromatics).

The following were made in an analogous manner from the appropriate starting materials :

1-Geranyl-2-methyl-4-nitrobenzimidazole $n_D$ = 1.5656, nmr peaks at δ 1.6, 1.7 and 1.8(s, 9H, 3 x Me); 2.0-(m, 4H, $CH_2$ $CH_2$); 2.6(s, 3H, N=C-Me); 4.7(d, 2H, $NCH_2$, 8Hz); 5.0(m, 2H, = CH x 2); 7.0-8.0(m, 3H, aromatics).

1-Geranyl-2-methyl-5(6)-nitrobenzimidazole $n_D$ = 1.5840, nmr peaks at δ 1.5,1.6 and 1.9(s,9H,3xMe); 2.1-(m,4H,$CH_2$-$CH_2$); 2.6(s,3H,N=C-Me); 4.7(d,2H,$NCH_2$8Hz); 5.0(m,2H,=CHx2); 7.0-8.2(m,3H,aromatics).

1-Geranyl-2,5,6-trimethylbenzimidazole $n_D$= 1.5534, n.m.r. peaks at δ1.6,1.7 and 1.8(s,9H,3xMe); 2.0-(m,4H,C <u>H</u>$_2$C<u>H</u>$_2$); 2.4(s,6H,2xMe); 2.5(s,3H,N=C-Me); 4.4 (d,2H, NC<u>H</u>$_2$,8Hz); 5.0(m,2H,=C<u>H</u>x2); 6.9 and 7.4-(s,2H,aromatics).

1-Geranyl-2,6,7-trimethylbenzimidazole $n_D$ = 1.5147, n.m.r. peaks at δ1.6,1.7 and 1.8(s,9H,3xMe); 2.0-(m,4H,C<u>H</u>$_2$C<u>H</u>$_2$); 2.4 and 2.6(s,6H,2xMe); 2.5(s,3H,N=C-Me);4.2(d,2H,NC<u>H</u>$_2$,8Hz); 5.0(m,2H,=C<u>H</u> x 2); 6.9-(m,2H, aromatics.

2,4-Dimethyl-1-geranylbenzimidazole $n_D$ = 1.5548, n.m.r peaks at δ1.5,1.6 and 1.8(s,9H,3xMe); 2.0-(m,4H,C<u>H</u>$_2$C<u>H</u>$_2$): 2.5(s,3H,N=C-Me); 2.6(s,3H,Me); 4.5(d,2H,$NCH_2$,8Hz); 5.0(m,2H,=CHx2); 7.0-(m,3H,aromatics).

1-Geranyl-5(6)-methoxy-2-methylbenzimidazole $n_D$ = 1.5512 n.m.r. peaks at δ 1.5,1.6 and 1.8(s,9H,3xMe); 2.0(m,4H,$CH_2CH_2$); 2.5(s,3H,N=C-Me); 3.5(s,3H, OMe); 4.5(d,2H,$NCH_2$,8Hz); 5.0(m,2H, =CHx2); 7.0-(m,3H,aromatics).

1-Geranyl-2-methyl-5(6)-trifluoromethylbenzimidazole $n_D$ = 1.5110, n.m.r. peaks at δ1.5,1.6 and 1.9 (s,9H,3xMe); 2.0(m,4H,$CH_2CH_2$); 2.5(s,3H,N=C-Me); 4.5(d,2H,$NCH_2$8Hz); 5.0(m,2H,=CH x 2); 7-7.9-(m,3H,aromatics).

4(7),6(5)-Dichloro-1-geranyl-2-methylbenzimidazole $n_D$ -1.5105 n.m.r. peaks at δ1.5,1.6 and 1.8(s,9H,3xMe); 2.0(m,4H,$CH_2CH_2$); 2.4(s,3H,N=C-Me); 4.4(d,2H,$NCH_2$,8Hz), 5.0(m,2H,=CHx2); 6.8-7.2(m,2H,aromatics).

4(7)-Chloro-1-geranyl-2-methyl-5(6)-trifluoro-methylbenzimidazole $n_D$ = 1.5222, n.m.r. peaks at δ1.5,1.6 and 1.8(s,9H,3xMe); 2.0(m,4H, $CH_2CH_2$; 2.4(s,3H, N=C-Me); 4.6(d,2H,$NCH_2$,8Hz); 5.0(m,2H,=CHx2); 7.1-7.3-(m,2H, aromatics.

5(6)-Ethoxy-1-geranyl-2-methyl-benzimidazole, n.m.r. peaks at δ 1.4(t,3H,C-$H_2CH_3$, 7Hz); 1.5,1.6 and 1.8 (s,9H,3xMe); 2.0 (m,4H,$CH_2CH_2$); 2.5(s,3H, N=CMe); 4.0 (q,2H, $OCH_2CH_3$,7Hz); 4.6(d,2H,$NCH_2$, 8Hz); 5.0(m, 2H, =CHX2); 6.6-7.6(m,3H, aromatics).

5(6)-Chloro-geranyl-2-methylbenzimidazole, n.m.r. peaks at δ 1.5, 1.6 and 1.8 (s,9H,3x Me); 2.0 (m,4H, $CH_2CH_2$); 4.6 (m, 2H, $NCH_2$); 5.0 (m,2H, =CHX2); 7.0 (m,3H, aromatics).

Methyl 1-geranyl-2-methyl-benzimidazole-7-carboxylate $n_D$ =1.5520, n.m.r. peaks at δ 1.5, 1.6 and1.8 (s,9H,3xMe); 2.0(m,4H, $CH_2CH_2$); 2.6 (s,3H, N=CMe); 3.8 (s,3H,OMe); 4.6(m,2H,$NCH_2$); 5.0(m,2H, =CHx2); 7.0-8.0 (m,3H, aromatics).

Replacing geranyl chloride with 1-bromo-7-methoxy-3,7-dimethyloct-2-ene or 1-bromo-7-ethoxy-3,7-dimethyloct-2-ene, the following compounds were made in an analogous manner:

(2E)-1-(7-Methoxy-3,7-dimethyloct-2-enyl)-2,4-dimethylbenzimidazole n.m.r. peaks at δ 0.7(s,6H); 1.0(m,3H); 1.4(m,4H); 2.1(s,3H); 2.2(s,3H); 2.7(m,3H); 4.2(m,2H), 4.7(m,1H); 6.6(m,3H,aromatics).

(2E)-1-(7-Methoxy-3,7-dimethyloct-2-enyl)-2,6,7-trimethylbenzimidazole n.m.r. peaks at δ1.1(s,6H); 1.4-(m,4H); 1.9(m,5H); 2.6(s,3H); 2.8(s,6H); 3.1(s,3H);4.8(m,2H); 5.1(m,1H); 6.9(s,2H),aromatics).

(2E)-5-Ethoxy-1-(7-Methoxy-3,7-dimethyloct-2-enyl)-2-methylbenzimididazole. n.m.r. peaks at δ1.1(s,6H); 1.4(m,6H); 1.8(m,4H); 2.5(s,3H); 3.1(s,3H; 4.0(q,2H); 4.6(m,2H); 5.1(m,1H); 7.0(m,3H, aromatics).

The following were made in a similar manner,

(2E,6E)-2,4-dimethyl-1-(3,7,11)-(trimethyldodeca-2,6,10-trienyl)benzimidazole. n.m.r. peaks at δ1.0(m,6H); 1.1(m,3H); 1.3(m,3H); 1.5(m,8H), 2.0(s,3H); 2.1(s,3H); 4.1(m,2H); 4.5(m,3H); 6.5(m,3H, aromatics).

(2E,6E)-2,6,7-Trimethyl-1-(3,7,11)-(trimethyldodeca-2,6,10-trienyl)benzimidazole. n.m.r. peaks at δ1,6(m,6H); 1.7(m,3H); 1.8(m,3H); 2.1(m,8H); 2.4(s,3H); 2.6(s,6H); 4.6(m,2H); 5.0(m,3H); 7.0(m,2H, aromatics).

(2E,6E)-5-Methoxy-2-methyl-1-(3,7,11-trimethyldodeca-2,6,10-trienyl)benzimidazole n.m.r. peaks at δ0.9-(m,6H); 1.0(m,3H); 1.1(m,3H); 1.3(m,8H); 1.8(s,3H); 3.1(s,3H); 3.9(m,2H); 4.4(m,3H); 6.5(m,3H,aromatics).

## EXAMPLE 8

### 4-Amino-1-geranyl-2-methylbenzimidazole

1-Geranyl-2-methyl-4-nitrobenzimidazole (320mg) was stirred in ethanol (15ml) under a nitrogen atmosphere. 5% palladium/charcoal ( 25mg) moistened with ethanol was added followed by the steady addition, over 5 minutes, of hydrazine hydrate (2.5ml). A further (25mg) of catalyst was added and the mixture brought to reflux for 0.75hr. The mixture was then allowed to stand at room temperature for 3 days. Following filtration over celite, the filtrate was concentrated and separated between ether and saturated sodium chloride. The organic layer was dried and concentrated to yield the title compound as a viscous yellow oil (250mg).

n.m.r. peaks at δ1.5, 1.6 and 1.7 (s,9H,3xMe); 2.0(m,4H,$CH_2CH_2$); 2.4(s,3H.N=CMe); 4.4(d,2H, $CH_2N$); 4.8 and 5.1(t,2H, 2x=CH); 6.5-7.0(m,3H, aromatics).

## EXAMPLE 9

### 4N-Acetylamino-1-geranyl-2-methylbenzimidazole

The amino benzimidazole from example 8 (100mg) was stirred in dry ether (10ml) containing pyridine (110mg). Acetyl chloride (56mg), in ether (0.5 ml) was added dropwise and the mixture allowed to stir for 2 hours at room temperature. Water (3ml) was added and the separated organic layer washed with saturated sodium chloride, dried and concentrated to yield a yellow oil (87mg). n.m.r. peaks at δ1.5, 1.6 and 1.7-(S,9H,3xMe), 2.0(m,4H,$CH_2CH_2$),2.3(s,3H,COMe); 2.4(s,3H,N=CMe); 4.4(d,2H,$CH_2N$); 4.5 and 5.1-(t,2H,2x=CH); 6.8-8.0(m,3H,aromatics).

## EXAMPLE 10

(5E)-7-(2-Methylbenzimidazole-1-yl)-1,1,5-trimethylhept-5-enyl-N-phenyl-carbamate

Phenyl isocyanate (0.5g) and lithium (50mgs) were added successively to a solution of 1-[(2E)-7-hydroxy-3,7-dimethylocta-2-enyl]-2-methyl-benzimidazole (1.0g) in dry benzene (60ml). The mixture was heated at reflux for 6h, evaporated to dryness and chromatographed on silica eluting with ethyl acetate to give the product (0.73g), m.pt = 132-3°C, $M^{+1}$ 406, n.m.r. peaks at δ1.4(s,6H,2xMe); 1.7(s,3H, = CMe); 1.9-(m,6H,$CH_2CH_2CH_2$); 2.6(s,3H, = NCMe); 4.6(d, 2H, $NCH_2$, 6Hz); 5.2(m,1H, = CH); 7.2(m,9H, aromatics).

EXAMPLE 11

2-Methylbenzimidazole was acylated with 1-chloro-5-isopropoxy-3-methyl-pent-2-ene (Swiss patent 600,761) under the usual conditions to give (E/Z)-1-(5-isopropoxy-3-methylpent-2-enyl)-2-methylbenzimidazole $n_D$ = 1.5215; n.m.r. peaks at δ 12(m,6H,2xMe); 1.6(m,1H,$CHMe_2$); 1.7 and 1.8(s,3H, = CMe); 2.6(s,3H, = NMe); 3.5(m,4H, $CH_2CH_2$); 4.7(m,2H,$NCH_2$); 5.3(m,1H, = CH); 7.2(m,4H,aromatics).

BIOLOGICAL ACTIVITY

A. Lethal activity against Insects

The activity of the compounds of the invention was tested by dissolving the compounds in acetone (5%) and then diluting in water: "Symperonic" (94.5%:0.5%) to give a water emulsion. The solution was then used to treat the following insects: *Musca domestica*, *Plutella xylostella*, *Myzus persicae* and *Tetranychus urticae*.

(I) *Musca domestica*:

20 Female *Musca domestica* were contained in a cardboard cyclinder with gauze over either end. A solution containing the compounds was sprayed onto the insects so enclosed and mortality assessed after 48 hrs at 25°C.

The following compounds were active at 1000 ppm:
1-Geranyl-2-methyl-4-nitrobenzimidazole
2-Chloro-1-geranylbenzimidazole
1-Geranyl-2,6,7-trimethylbenzimidazole
1-Geranyl-5(6)-methoxy-2-methylbenzimidazole
4-Amino-1-geranyl-2-methylbenzimidazole

The following compounds were active at 200 ppm
1-Geranyl-2,4-dimethyl-benzimidazole
(2E)-1-(7-Methoxy-3,7-dimethyloct-2-enyl)-2,4,5-trimethylbenzimidazole
(2E)-1-(7-Methoxy-3,7-dimethyloct-2-enyl)-2,4-dimethylbenzimidazole

(II) The activity of compounds of the invention against unanaesthetised female *Musca domestica* (WRL strain), was also demonstrated by the topical application to the test insect of a solution of the compound under test in cellosolve. Mortality was assessed after 10 mins, 1 hr, 24 hrs and 48 hrs.

Compounds which had an $LD_{50}$ of <10μg/fly include:
1-Geranyl-2-methyl-4-nitrobenzimidazole
1-Geranyl-2-methyl-5(6)-nitrobenzimidazole
2-Chloro-1-geranylbenzimidazole
1-Geranyl-2,6,7-trimethylbenzimidazole
1-Geranyl-2,4-dimethylbenzimidazole
1-Geranyl-5(6)-methoxy-2-methylbenzimidazole
4-Amino-1-geranyl-2-methylbenzimididazole
(2E)-1-(7-Methoxy-3,7-dimethyloct-2-enyl)-2,4,5-trimethylbenzimidazole
(2E)-1-(7-Methoxy-3,7-dimethyloct-2-enyl)-2,4-dimethylbenzimidazole

(5E)-7-(2-Methylbenzimidazole-1-yl)-1,1,5-trimethylhept-5-enyl-N-phenylcarbamate
(E/Z)-1-(5-Isopropoxy-3-methylpent-2-enyl)-2-methylbenzimidazole
1-[(2E)-6-Methoxyimino-3-methylhex-2-enyl]-2-methylbenzimidazole.
(2E)-2-Methyl-1-(3-methylocta-2,6-dienyl)benzimidazole.

(III) Plutella xylostella:

7 Plutella larvae were sprayed with the solution containing the compound and added to a chinese cabbage leaf which had been similarly sprayed and left to dry. Mortality was assessed after 2-days at 25°C.

(2E)-1-(7-Methoxy-3,7-dimethyloct-2-enyl)-2,4,5-trimethylbenzimidazoleazole was active at 1000 ppm.

(IV) Myzus persicae

10 adult Myzus were placed on a leaf disc of chinese cabbage. 24 hours later the disc was sprayed with the solution containing the compound. Mortality was assessed after 2 days at 25°C.

The following compounds were active on Myzus persicae at 1000 ppm:
1-Geranyl-2-methyl-5(6)-nitrobenzimidazole
1-Geranyl-2,6,7-trimethylbenzimidazole
1-Geranyl-5(6)-methoxy-2-methylbenzimidazole
2-Chloro-1-geranylbenzimidazole

The following compounds were active at 200 ppm:
1-Geranyl-2-methyl-4-nitrobenzimidazole
1-Geranyl-2,4-dimethylbenzimidazole
(2E)-1-(7-Methoxy-3,7-dimethyloct-2-enyl)-2,4,5-trimethylbenzimidazole
(2E)-1-(7-Methoxy-3,7-dimethyloct-2-enyl)-2,4-dimethylbenzimidazole

(V) Tetranychus urticae

Leaf discs of infested french bean were sprayed with the solution containing the compound. Mortality was assessed after 2 days at 25°C.

(2E)-1-(7-Methoxy-3,7-dimethyloct-2-enyl)-2,4-dimethylbenzimidazole was active against Tetranychus urticae at 200 ppm.

B. Antifungal Testing

The antifungal activity of compounds of the invention was determined using a primary soft agar screen. The concentration dependent inhibition of hyphae growth was determined for Coriolusversicolor, Coniophora putena, Aureobasidium pullulans and Chaetomium globosum.

The following compounds had an $IC_{50}$ 30 ppm
5(6)-Chloro-1-geranyl-2-methylbenzimidazole
1-Geranyl-2-methyl-4-nitrobenzimidazole
1-Geranyl-2,6,7-trimethylbenzimidazole
1-Geranyl-2,5,6-trimethylbenzimidazole
1-Geranyl-2,4-dimethylbenzimidazole
1-Geranyl-5(6)-methoxy-2-methylbenzimidazole
1-Geranyl-5(6)-ethoxy-2-methylbenzimidazole
Methyl 1-geranyl-2-methylbenzimidazole-7-carboxylate
1-Geranyl-4,6-dichloro-2-methylbenzimidazole
4-Amino-1-geranyl-2-methylbenzimidazole
(2E)-1-(7-Methoxy-3,7-dimethyloct-2-enyl)-2,6,7-trimethylbenzimidazole
(2E)-1-(7-Methoxy-3,7-dimethyloct-2-enyl)-2,4-dimethylbenzimidazole
(5E)-7-(2-Methylbenzimidazole-1-yl)-1,1,5-trimethylhept-5-enyl-N-phenylcarbamate
2-Methyl-5(6)-methoxy-1-[(2E,6E)-3,7,11-trimethyldodeca-2,6,10-trienyl]benzimidazole
2,4-Dimethyl-1-[(2E,6E)-3,7,11-trimethyldodeca-2,6,10-trienyl]benzimidazole.

1-[3-Methylhepta-2,6-dienyl]-2-methylbenzimidazole
1-[Hepta-2,6-dienyl]-2-methylbenzimidazole
(2E)-2-Methyl-1-(3-methylocta-2,6-dienyl)benzimidazole
2-Methyl-1-[(2E)-3-methyl-6-benzyloxyiminohex-2-enyl]benzimidazole
1-[(2E)-7,7-Dichloro-3-methylhepta-2,6-dienyl]-2-methylbenzimidazole

C. Mammalian Toxicity

1-(2E)-6-Methoxyimino-3-methylhex-2-enyl-2-methylbenzimidazole and 1-geranyl-2,6,7-trimethylbenzimidazole have an $LD_{50}$ value of approximately 200 mg when given orally to mice (Charles River CD1).

Formulations

1. Emulsifiable Concentrate

| | |
|---|---|
| Compound of formula (I) | 10.00 |
| Ethlyan KEO | 20.00 |
| Xylene | 67.50 |
| Butylated Hydroxyanisole | 2.50 |
| | 100.00 |

2. Wettable Powder

| | |
|---|---|
| Compound of formula (I) | 25.0 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

3. Dust

| | |
|---|---|
| Compound of formula (I) | 0.50 |
| Butylated Hydroxyanisole | 0.10 |
| Talc | 99.40 |
| | 100.00 |

4. Bait

| | |
|---|---|
| Compound of formula (I) | 40.25 |
| Icing Sugar | 59.65 |
| Butylated hydroxy toluene | 0.10 |
| | 100.00 |

5. Lacquer

| | |
|---|---|
| Compound of formula (I) | 2.5 |
| Resin | 5.0 |
| Butylated Hydroxy anisole | 0.5 |
| High aromatic white spirit | 92.0 |
| | 100.00 |

6. Aerosol

| | |
|---|---|
| Compound of formula (I) | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

7. Spray

| | |
|---|---|
| Compound of formula (I) | 0.1 |
| Butylated Hydroxy anisole | 0.1 |
| Xylene | 10.0 |
| Odourless Kerosene | 89.8 |
| | 100.00 |

8. <u>Potentiated Spray</u>

| | |
|---|---|
| Compound of formula (I) | 0.1 |
| Piperonyl Butoxide | 0.5 |
| Butylated Hydroxyanisole | 0.1 |
| Xylene | 10.1 |
| Odourless Kerosene | 89.2 |
| | 100.0 |

## Claims

1) A compound of the formula (I)

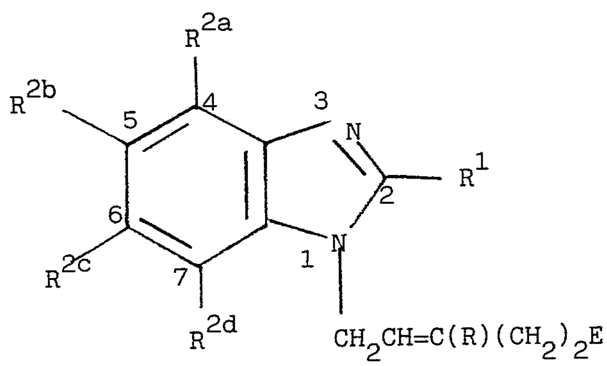

(I)

or an acid addition salt thereof, wherein R is hydrogen or $C_{1-4}$alkyl, $R^1$ is chlorine or methyl optionally substituted by one or two fluorine, $R^{2a}$ to $R^{2d}$ are the same or different and are chosen from the group comprising hydrogen, chlorine, bromine, fluorine, $C_{1-4}$alkyl optionally substituted by fluorine or chlorine atoms, $C_{1-4}$alkoxy, $C_{1-4}$ acyl, $C_{1-4}$ alkoxycarbonyl, $C_{1-4}$alkylthio, nitro, cyano, amino optionally substituted by $C_{1-4}$ alkyl or $C_{1-4}$ acyl; E is $C_{6-10}$ aryl or aralkyl, a group $CH_2C(Me)_2OR^3$ wherein $R^3$ is hydrogen, $C_{1-4}$ acyl, $C_{1-4}$ alkyl or $C_{2-8}$ carbamyl, a group $CH=C(Me)CH_2CH_2CH=CMe_2$, a group $-CH\overset{O}{—}CMe_2$, a group $OCHR^4R^5$ wherein $R^4$ and $R^5$ are the same or different and each is methyl or ethyl, a group $CH=CR^6R^7$ wherein $R^6$ is hydrogen, chlorine, bromine, fluorine, $C_{1-4}$ alkyl, $C_{1-8}$ alkoxycarbonyl, $C_{1-4}$ alkynyl or $C_{1-8}$ acyl and $R^7$ is hydrogen, chlorine, bromine, fluorine or $C_{1-4}$ alkyl, or a group $CH=X-Y-R^8$ wherein X is N or $C(R^9)$ wherein $R^9$ is hydrogen or $C_{1-6}$alkyl, Y is oxygen, sulphur or a group $NR^{10}$ wherein $R^{10}$ is $C_{1-8}$hydrocarbyl, $R^8$ is $C_{1-4}$ acyl or $C_{1-10}$ hydrocarbyl optionally substituted by $C_{1-4}$ alkyl, $C_{1-4}$ alkoxy, $C_{1-4}$ acyl or halogen, or, when X is nitrogen, $R^8$ may be hydrogen;
provided that at least of of $R^{2a}$ to $R^{2d}$ is other than hydrogen when $R^1$ is methyl or chlorine except when E is $OCHR^4R^5$, $C_{6-10}$ aryl or aralkyl, $CH=CR^6R^7$ or $CH=XYR^8$ or $R^3$ is $C_{2-8}$ carbamyl and that $R^{2c}$ is not methyl when $R^1$ is methyl except when E is $CH=CR^6R^7$ or $CH=XYR^8$, and provided that the compound of formula (I) is not 1-geranyl-2-methylbenzimidazole.

2. A compound of the formula (I) according to claim 1 wherein E is $C_{6-10}$ aryl or arlkyl, a group $CH_2C(Me_2)OR^3$, a group $CH = CMeCH_2CH_2CH = CMe_2$, a group $CH = CMe_2$, a group $-CH\overset{O}{—}CMe_2$ or a group $OCH\text{-}R^4R^5$ wherein $R^4$ and $R^5$ are the same or different and each is methyl or ethyl, wherein $R^3$ is hydrogen, $C_{1-4}$ acyl, $C_{1-4}$ alkyl, or $C_{2-8}$ carbamyl.

3) A compound of the formula (I) according to claim 1 wherein E is a group $CH = XYR^8$ or $CH = CR^6R^7$ wherein X is nitrogen or $C(R^9)$ wherein $R^9$ is $C_{1-6}$ alkyl or hydrogen, Y is oxygen, sulphur or a group $NR^{10}$ wherein $R^{10}$ is $C_{1-8}$ hydrocarbyl; $R^8$ is $C_{1-4}$ acyl or $C_{1-10}$hydrocarbyl optionally substituted by $C_{1-4}$ alkyl, $C_1$-

$_4$alkoxy, $C_{1-4}$acyl or hydrogen, or, when X is nitrogen, alternatively $R^8$ is hydrogen; $R^6$ is chosen from hydrogen, chlorine, bromine, fluorine, $C_{1-4}$ alkyl, $C_{1-8}$ alkoxycarbonyl, $C_{1-4}$ alkenyl and $C_{1-8}$ acyl; and $R^7$ is chosen from hydrogen, chlorine, bromine, fluorine and $C_{1-4}$ alkyl.

4. A compound of the formula (I) according to claim 1 wherein E is $CH_2C(Me)_2OR^{3a}$ wherein $R^{3a}$ is methyl, ethyl or acetyl, $CH = CH(Me)_2$, $CH = CH(Me)CH_2CH_2CH=CHMe$ or $CH = NOR^8$ wherein $R^8$ is methyl or hydrogen.

5. A compound of the formula (I) according to any one of claims 1 to 4 wherein $R^1$ is hydrogen or methyl and is methyl.

6. A compound of the formula (I) according to any one of claims 1 to 5 wherein one or $R^{2a}$ to $R^{2d}$ is chosen from nitro, methyl, methoxy, ethoxy and chlorine.

7. A formulation for the control of pests comprising a compound of the formula (I) according to any one of claims 1 to 6 in admixture with a pesticidally acceptable carrier.

8. A compound of the formula (I) according to any of claims 1 to 6 for the control of insect, acarine, helminth or fungal pests.

9. A method for the control or eradication of insect pests comprising the application of an effective amount of a compound of the formula (I) according to any one of claims 1 to 6 to the insect or to a locus in the vicinity of the insect.

10. A method for the control or eradication of acarine pests comprising the application of an effective amount of a compound of the formula (I) according to any one of claims 1 to 6 to the acarine or to a locus in the vicinity of the acarine.

11. A method for the control or eradication of fungal pests comprising the application of an effective amount of a compound of the formula (I) according to any one of claims 1 to 6 to the fungus.

12. A process for the preparation of a compound of the formula (I) which comprises (a) the reaction of a compound of the fomrula (II):

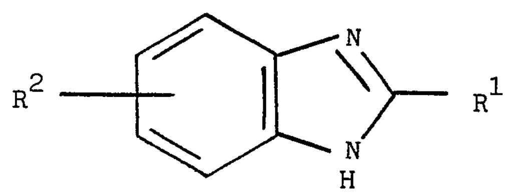

(II)

Wherein $R^1$ is as defined in claim 1 and $R^2$ represents $R^{2a}$ to $R^{2d}$, with an alkylating agent of the formula

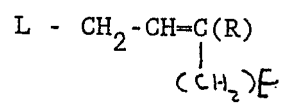

wherein L is a leaving group, and R and E are as defined in claim 1, and
b) when E is $CH = NOR^8$ or $CH = CR^6R^7$ the reaction of a compound of formula (III) :

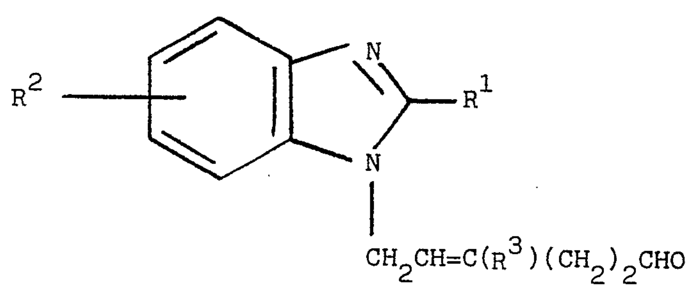

(III)

wherein $R^1$,$R^2$ and R are as defined in claim 12a, with the appropriate hydroxylamine or Wittig type reagent, and

c) thereafter optionally converting one compound of the formula (I) into another compound of the formula (I).

Claims for the following contracting states: GR and ES

1. A process for the preparation of compound of the formula (I)

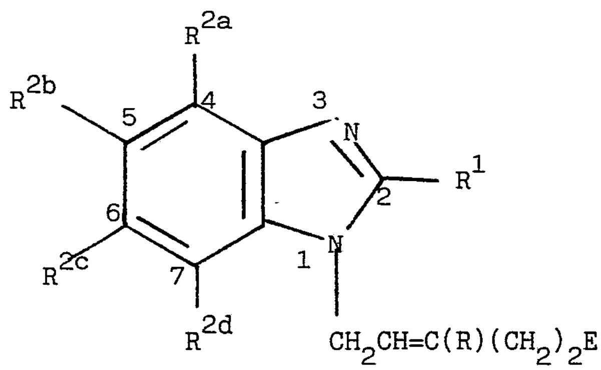

(I)

or an acid addition salt thereof, wherein R is hydrogen or $C_{1-4}$alkyl, $R^1$ is chlorine or methyl optionally substituted by one or two fluorine, $R^{2a}$ to $R^{2d}$ are the same or different and are chosen from the group comprising hydrogen, chlorine, bromine, fluorine, $C_{1-4}$ alkyl optionally substituted by fluorine or chlorine atoms, $C_{1-4}$ alkoxy, $C_{1-4}$ acyl, $C_{1-4}$ alkoxycarbonyl, $C_{1-4}$alkylthio, nitro, cyano, amino optionally substituted by $C_{1-4}$ alkyl or $C_{1-4}$ acyl; E is $C_{6-10}$ aryl or aralkyl, a group $CH_2C(Me)_2OR^3$ wherein $R^3$ is hydrogen, $C_{1-4}$ acyl, $C_{1-4}$ alkyl or $C_{2-8}$ carbamyl, a group $CH=C(Me)CH_2CH_2CH=CMe_2$, a group - 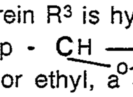, a group $OCHR^4R^5$ wherein $R^4$and $R^5$ are the same or different and each is methyl or ethyl, a group $CH=CR^6R^7$ wherein $R^6$ is hydrogen, chlorine, bromine, fluorine,$C_{1-4}$ alkyl, $C_{1-8}$ alkoxycarbonyl, $C_{1-4}$alkynyl or $C_{1-8}$ acyl and $R^7$ is hydrogen, chlorine, bromine, fluorine or $C_{1-4}$ alkyl, or a group $CH=X\text{-}Y\text{-}R^8$ wherein X is N or $C(R^9)$ wherein $R^9$ is hydrogen or $C_{1-6}$ alkyl, Y is oxygen, sulphur or a group $NR^{10}$ wherein $R^{10}$ is $C_{1-8}$ hydrocarbyl, $R^8$ is $_{1-4}$ acyl or $C_{1-10}$ hydrocarbyl optionally substituted by $C_{1-4}$ alkyl, $C_{1-4}$ alkoxy, $C_{1-4}$ acyl or halogen, or, when X is nitrogen, $R^8$ may be hydrogen; provided that at least one or $R^{2a}$ to $R^{2d}$ is other than hydrogen when $R^1$ is methyl or chlorine except when E is $OCHR^4R^5$, $C_{6-10}$ aryl or aralkyl, $CH=CR^6R^7$ or $CH=XYR^8$ or $R^3$ is $C_{2-8}$ carbamyl and that $R^{2c}$ is not methyl when $R^1$ is methyl except when E is $CH=CR^6R^7$ or $CH=XYR^8$, and other than 1-geranyl-2-methyl-benzimidazole; which comprises (a) the reaction of a compound of the formula (II) :

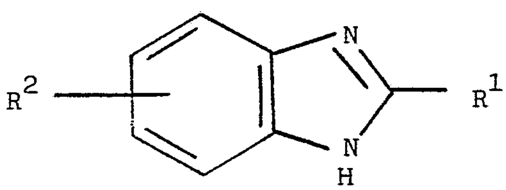

(II)

wherein $R^1$ is as defined in claim 1 and $R^2$ represents $R^{2a}$ to $R^{2d}$, with an alkylating agent of the formula

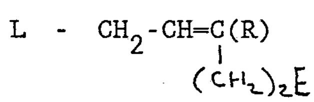

wherein L is a leaving group, and R and E are as defined in claim 1, and
b) when E is $CH = NOR^8$ or $CH = CR^6R^7$ the reaction of a compound of formula (III) :

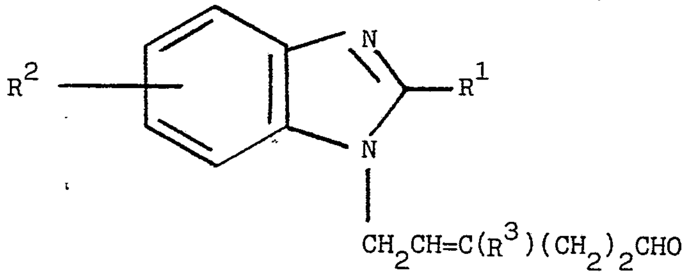

(III)

wherein $R^1$, $R^2$ and R are as defined in claim 1 a, with the appropriate hydroxylamine or Wittig type reagent, and

c) thereafter optionally converting one compound of the formula (I) into another compound of the formula (I).

2. A process according to claim 1 for the preparation of a compound of the formula (I), wherein E is $C_{6-10}$ aryl or aralkyl, a group $CH_2C(Me_2)OR^3$, a group $CH = CMeCH_2CH_2CH = CMe_2$, a group $CH = CMe_2$, a group - 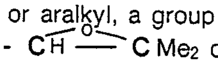 or a group $OCH\text{-}R^4R^5$ wherein $R^4$ and $R^5$ are the same or different and each is methyl or ethyl, wherein $R^3$ is hydrogen, $C_{1-4}$ acyl, $C_{1-4}$ alkyl, or $C_{2-8}$ carbamyl.

3. A compound of the formula (I) according to claim 1 for the preparation of a compound of the formula (I) wherein E is a group $CH = XYR^8$ or $CH = CR^6R^7$ wherein X is nitrogen or $C(R^9)$ wherein $R^9$ is $C_{1-6}$ alkyl or hydrogen, Y is oxygen, sulphur or a group $NR^{10}$ wherein $R^{10}$ is $C_{1-8}$hydrocarbyl; $R^8$ is $C_{1-4}$ acyl or $C_{1-10}$ hydrocarbyl optionally substituted by $C_{1-4}$ alkyl, $C_{1-4}$alkoxy, $C_{1-4}$ acyl or halogen, or, when X is nitrogen, alternatively $R^8$ is hydrogen; $R^6$ is chosen from hydrogen, chlorine, bromine, fluorine, $C_{1-4}$ alkyl, $C_{1-8}$alkoxycarbonyl, $C_{1-4}$ alkenyl and $C_{1-8}$ acyl; and $R^7$ is chosen from hydrogen, chlorine, bromine, fluorine and $C_{1-4}$ alkyl.

4. A process according to claim 1 for the preparation of a compound of the formula (I) wherein E is $CH_2C(Me)_2OR^{3a}$ wherein $R^{3a}$ is methyl, ethyl or acetyl, $CH = CH(Me)_2$, $CH = CH(Me)CH_2CH_2CH = CHMe$ or $CH = NOR^8$ wherein $R^8$ is methyl or hydrogen.

5. A process according to any of claims 1 to 4 for the preparation of a compound for the formula (I) wherein $R^1$ is hydrogen or methyl and is methyl.

6. A process according to any one of claims 1 to 5 for the preparation of a compound of the formula (I) wherein one of $R^{2a}$ to $R^{2d}$ is chosen from nitro, methyl, methoxy, ethoxy and chlorine.

7. A formulation for the control of pests comprising a compound of the formula (I) according to any one of claims 1 to 6 in admixture with a pesticidally acceptable carrier.

8. A compound of the formula (I) according to any of claims 1 to 6 for the control of insect, acarine, or helminth or fungal pests.

9. A method for the control or eradication of insect pests comprising the application of an effective amount of a compound of the formula (I) according to any one of claims 1 to 6 to the insect or to a locus in the vicinity of the insect.

10. A method for the control or eradication of acarine pests comprising the application of an effective amount of a compound of the formula (I) according to any one of claims 1 to 6 to the acarine or to a locus in the vicinity of the acarine.

11. A method for the control or eradication of fungal pests comprising the application of an effective amount of a compound of the formula (I) according to any one of claims 1 to 6 to the fungus.

European Patent Office

# EUROPEAN SEARCH REPORT

Application Number

EP 87 30 8918

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 102, no. 11, 18th March 1985, page 576, abstract no. 95645a, Columbus, Ohio, US; & JP-A-59 181 263 (NIHON NOHYAKU CO., LTD) 15-10-1984 | | C 07 D 235/08<br>C 07 D 235/24<br>C 07 D 405/06<br>A 01 N 43/52 |
| A | CHEMICAL ABSTRACTS, vol. 101, no. 1, 2nd July 1984, pages 612-613, abstract no. 7158v, Columbus, Ohio, US; & JP-A-59 21 671 (KYUSHU UNIVERSITY) 03-02-1984 | | |
| A | CHEMICAL ABSTRACTS, vol. 101, no. 15, 8th October 1984, page 301, abstract no. 125702r, Columbus, Ohio, US; E. COHEN et al.: "The use of a Tribolium chitin synthetase assay in studying the effects of benzimidazoles with a terpene moiety and related compounds", & AGRIC. BIOL. CHEM. 1984, 48(6), 1617-20 | | TECHNICAL FIELDS SEARCHED (Int. Cl.4) |
| D,A | CHEMICAL ABSTRACTS, vol. 97, no. 11, 13th September 1982, page 288, abstract no. 87004d, Columbus, Ohio, US; E. KUWANO et al.: "Insecticidal benzimidazoles with a terpenoid moiety", & AGRIC. BIOL. CHEM. 1982, 46(6), 1715-16 | | C 07 D 235/00<br>C 07 D 405/00<br>A 01 N 43/00 |

The present search report has been drawn up for all claims

| Place of search | Date of completion of the search | Examiner |
|---|---|---|
| THE HAGUE | 15-01-1988 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)